(19) Europäisches Patentamt
European Patent Office
Office européen des brevets

(11) **EP 2 581 386 A1**

(12) **EUROPEAN PATENT APPLICATION**

(43) Date of publication:
**17.04.2013 Bulletin 2013/16**

(51) Int Cl.:
***C07K 14/775*** (2006.01)

(21) Application number: **11185180.4**

(22) Date of filing: **14.10.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Philip Morris Products S.A.
2000 Neuchâtel (CH)**

(72) Inventors:
• **Steffen, Yvonne
50161 Cologne (DE)**

• **Schüller, Jutta
51145 Cologne (DE)**
• **Grandjean, René
53111 Bonn (DE)**
• **Hengstermann, Arnd
50733 Cologne (DE)**

(74) Representative: **Vossius & Partner
Siebertstrasse 4
81675 München (DE)**

(54) **Isolation of LDL from serum or plasma samples**

(57)    The present invention provides an easy and time efficient method for obtaining concentrated LDL fractions from relatively small volumes of plasma/serum. In particular, the method comprises the steps of (i) applying a single discontinuous density gradient ultracentrifugation (ii) collecting the LDL containing faction from the sample after centrifugation and (iii) concentrating the LDL containing fraction.

**Description**

The present invention provides an easy and time efficient method for obtaining concentrated low density lipoprotein (LDL) fractions from relatively small sample volumes such as plasma or serum. In particular, the method comprises the steps of (i) applying discontinuous density gradient ultracentrifugation (ii) collecting the LDL containing faction from the sample after centrifugation and (iii) concentrating the LDL containing fraction.

Recent evidence suggests that the presence of low density lipoprotein (LDL) is associated with increased risk of developing coronary artery disease. In one hypothesis, LDL particles, which are known to transport cholesterol into the artery wall, are believed to be retained there by arterial proteoglycans and to attract macrophages that engulf the LDL particles and start the formation of plaques. Over time vulnerable plaques rupture, activate blood clotting and produce arterial stenosis, which may result in heart attack, stroke, and peripheral vascular disease symptoms and major debilitating events.

In order to further elucidate the role of LDL in the development of arteriosclerosis and other diseases, isolation of reasonable amounts of LDL which are essentially free of lipid hydroperoxide contaminations, are required. Current methods to fractionate LDL from serum or plasma samples are usually very time-consuming and/or technically demanding. Moreover, conventional LDL preparations often yield unstable LDL fractions which are very prone to oxidation during the long isolation procedure, handling, and storage. Hence they are usually not usable for further downstream applications (e.g., comparison of LDL and oxLDL in *in vitro* and *in vivo* assays, as a biomarker, etc.)

Lipoproteins have been commonly isolated by ultracentrifugation on the basis of their hydrated density characteristics. Most of the proposed methods are very time consuming and laborious, require a large volume of plasma, and may lead to damage of particles. It has been reported that prolonged ultracentrifugation could result in a partial degradation of apolipoprotein B (apoB) (Kleinveld et. al. 1992).

The most common and widely used method for isolation of LDL is a two-step sequential ultracentrifugation process with a total run duration of about 48h (Havel and Eder 1955, Kleinveld et. al. 1992). However, these traditional and time-consuming methodologies (up to 48 h) yield LDL preparations that contain lipid hydroperoxides which are thought to play an important role in oxidative modification of LDL. Hence, conventional LDL preparations are often unstable and very prone to oxidation during the long isolation procedure, handling, and storage.

Viera et al (1996) report a process for isolating low density lipoproteins in a concentrated fraction free from water-soluble plasma antioxidants. This method comprises discontinuous gradient density centrifugation followed by a simultaneous purification and concentration step using ultrafiltration through a collodium bag under nitrogen.

There is therefore a need to for improved, less complex and time-consuming LDL isolation procedures, which allow preparation of highly concentrated and ready to use LDL fractions from samples obtained from single donor subjects (human or rodents), in a very short procedure duration.

This need could be satisfied by providing the method as disclosed and claimed herein. The method according to the invention has various advantages over the state of the art procedures. It is a very time-efficient method for obtaining concentrated LDL fractions from relatively small sample volumes such as plasma or serum. The obtained LDL fraction contains no measurable lipid peroxidation, which is of high importance for *in vitro and in vivo* downstream studies. LDL preparations obtained by the method according to the invention will thus also be suitable for use in *in vitro* cell culture assays.

The present invention provides a method for isolating LDL from a sample, particularly a plasma or serum sample, which method comprises the steps of (i) applying discontinuous density gradient ultracentrifugation to the sample, (ii) collecting the LDL containing faction or fractions from the sample after ultracentrifugation, and (iii) concentrating the LDL containing fraction(s) by, for example, applying gelfiltration and/or by a desalting column.

In the method of the invention and as described herein, the discontinuous density gradient may be established by overlaying the LDL-containing sample with a solution of relatively high density comprising one or more alkali metal salts ("heavy salt solution"), particularly halides, such as NaCl and/or KBr.

The one or more alkali metal salts may each be independently present in the heavy salt solution at a molar concentration of between 2.5 and 3.0.

In another embodiment, the heavy salt solution used in the method according to the invention and as described herein, further comprises a chelating agent such as, for example, EDTA.

In one embodiment, the method according to the invention and as described herein comprises an ultracentrifugation step, wherein the ultracentrifugation is carried out at 49'000g - 110'000g, particularly at 77'000g to 103'000g and more particularly at 96'500g.

In a specific embodiment of the invention, the ultracentrifugation is carried out in a Beckman SW41 Ti rotor at a speed (measured in revolutions per minute) of between 20'000 - 30'000 rpm, particularly at a speed of 25'000 - 29'000 for 2 - 2.5 h, particularly at a speed of 28'000 rpm for 2.1 hours. Accordingly, based on the equation, where Relative Centrifugal Field (RCF) is measured in g (standard acceleration of gravity) and r is the radius of the centrifuge rotor,

$$RCF = 1.12r \times (rpm/1000)^2$$

the applicable RCF ranges from 49'000g to 111'000g, particularly from 77'000g to 103'000g and more particularly at 96'500g. The calculation of the applicable RCF is based on the average radius of the SW41 rotor which is 110mm, while the minimum radius is 67 mm and the maximum radius is 153 mm.

In one embodiment, the method according to the invention and as described herein comprises an ultracentrifugation step, wherein the ultracentrifugation is carried out at a temperature below 10° C, particularly at a temperature of between 2° C and 10° C, particularly of between 3° C and 5° C, but especially at 4° C.

In one embodiment, the method according to the invention and as described herein comprises only a single centrifugation step, particularly only one ultracentrifugation step.

In one embodiment, the method according to the invention and as described herein comprises an LDL concentration step for the LDL fraction collected after ultracentrifugation, which is carried out under normal atmospheric conditions. The method according to the invention does not require working under nitrogen or another inert gas.

Accordingly, in one embodiment, the method according to the invention and as described herein does not comprise use of positive fluid pressure or use of nitrogen to concentrate one or more LDL-containing fractions.

In one embodiment, the method according to the invention and as described herein leads to the isolation of a LDL fraction, which does not contain any detectable lipid hydroperoxide contaminations.

Accordingly, in one embodiment of the invention, a concentrated LDL solution is provided which does not contain a measureable lipid peroxidation and/or is essentially free of hydroperoxide contaminations.

In one embodiment, the method according to the invention and as described herein comprises the additional step of storing the concentrated LDL solution at a temperature of between 1° and 10°C, particularly of between 2°C and 6°C, but especially at 4°C, in the dark, and, in one embodiment, under liquid nitrogen, argon or an inert gas.

The foregoing description will be more fully understood with the reference to the following Examples. Such Examples are, however, exemplary methods of practicing the present invention and are not intended to limit the scope of the invention.

EXAMPLE

1. *Sample preparation for centrifugation*

The plasma or serum solution was distributed into 12-ml polycarbonate centrifuge tubes and a discontinuous density gradient was made by overlaying the plasma solution (0.9 ml) with 1,93 ml heavy salt solution (2,62 M NaCl, 2,97 M KBr, 0,27 pM EDTA) and approximately 10 ml phosphate buffered saline (Sigma).

2. *Ultracentrifugation*

The tubes were ultracentrifuged in a Beckman ultracentrifuge equipped with a SW 41 Ti rotor, at 28000 rpm for 2,10 h at 4°C (acceleration 7, deceleration 4).

3. *Isolation and Concentration of LDL fraction.*

*3.1 Method 1*

After centrifugation, the tubes were carefully removed from the rotor and placed in the vertical position. The VLDL fraction appears as a white, heavily light scattering band at the meniscus. The yellow-orange LDL fraction stayed in the upper half of the tube. The LDL fraction was collected by suction using a long-stem Pasteur pipette. The pipette was introduced through the VLDL fraction with a small air bubble at the end to avoid suction of materials other than the band of LDL.

The LDL fraction was concentrated and simultaneously desalted by a PD desalting column (Millipore). Thereafter, the LDL solution was stored at 4°C in the dark for up to 8 days under liquid nitrogen .

*3.2 Method 2*

After centrifugation, the tubes are carefully removed from the buckets with forceps. The upper layer is removed with a Pasteur pipette without disturbing the orange-colored LDL layer.

The LDL-containing fraction is cautiously transferred with a new Pasteur pipette into a new tube and pooled by adding samples from different tubes to reach a volume of at least 3 ml.

Columns (Gelfiltrationssäule PD-10, Amersham) are prepared for gelfiltration. The column is transfixed in a support frame, the tip of the column is cut and beaker glass positioned under the column. The column is equilibrated with 20 ml PBS.

The isolated LDL fraction (2.5 ml) is pipetted to the column and the first eluate fraction discarded.

A falcon tube is positioned under the columns and LDL eluted from the column with 3.5 ml PBS buffer to the falcon.

The eluate can be stored for up to one week at 4 °C $\pm$ 1 °C in the dark under liquid nitrogen.

4. *Analysis of LDL fraction*

The protein concentration was determined according to the method of Lowry et al (1951) with a ready to use Lowry kit according to the suppliers instructions.

The LDL obtained under these conditions contained no measurable lipid peroxidation.

REFERENCE LIST

Chung, B. H., J. P. Sergrest, M. J. Ray, J. D. Brunzell, J. E. Hokanson, R. M. Krauss, K. Beaudrie, and J. T. Cone. 1986. Single vertical spin density gradient ultracentrifugation. Methods Enzymol. 128: 181-209

Havel, R.J., Eder, H.A., and Bragdon, J.H. 1955. The distribution and chemical composition of ultracentrifugally separated lipoproteins in human serum. J. Clin. Invest. 34: 1345-1353

Kleinveld H. A.; Hak-Lemmers H. L. M.; Stalenhoef A. F. H.;Demacker P. N. M 1992. Improved measurement of low-density lipoprotein susceptibility to copper-induced oxidation: Application of a short procedure for isolating low-density lipoprotein. Clin Chem 38, 2066-2072

Lowry, 0. H., N. J. Rosebrough, A. L. Farr, and R. J. Randall. 1951. Protein measurement with the Folin phenol reagent. J. Biol. Chem. 193: 265-275.

Mironova M;. Virella G; Lopes-Virella M. F. 1996 Isolation and Characterization of Human Antioxidized LDL Autoantibodies. Arteriosclerosis, Thrombosis, and Vascular Biotogy. 16:222-229

Vieira V. Otilia, Laranjinha, João A. N., Madeira M. C. Vitor, and Almeida M. Leonor, 1996, "Rapid Isolation of Low Densitiy Lipoproteins in a Concentrated Fraction Free From Water-soluble Plasma Antioxidants, Journal of Lipid Resarch, Volume 37; 2715-2721.

**Claims**

1. A method for isolating low density lipoprotein (LDL) from a LDL-containing sample, which method comprises the steps of (i) applying discontinuous density gradient ultracentrifugation to the sample, (ii) isolating one or more LDL-containing fraction(s) from the sample after ultracentrifugation, and (iii) concentrating the LDL-containing fraction(s) by applying gelfiltration and/or by a desalting column.

2. The method of claim 1, wherein said LDL-containing sample is plasma or serum.

3. The method of any one of the preceding claims, wherein the discontinuous density gradient is established by overlaying the LDL-containing sample with a heavy salt solution comprising one or more alkali metal salts.

4. The method according to claim 3, wherein said alkali metal salt is NaCl and/or KBr.

5. The method according to claims 3 or 4, wherein the one or more alkali metal salts are each independently present at a molar concentration of between 2.5 and 3.0.

6. The method according to any one of the preceding claims, wherein said heavy salt solution further comprises EDTA.

7. The method of any one of the preceding claims, wherein the ultracentrifugation is carried out at 49'000g - 110'000g.

8. The method of any one of the preceding claims, wherein the ultracentrifugation is carried out at 77'000g - 103'000g four 2 - 2.5 h.

9. The method of any one of the preceding claims, wherein the ultracentrifugation is carried out at 20'000rpm - 30'000rpm for 2 - 2.5 h in a rotor with an average radius of 110mm.

10. The method according to any one of the preceding claims, wherein the ultracentrifugation is carried out at a temperature below 10° C.

11. The method according to any one of the preceding claims, wherein the isolated LDL does not contain any detectable lipid hydroperoxide contaminations.

12. The method according to any one of the preceding claims, which comprises only a single ultracentrifugation step.

13. The method according to any one of the preceding claims, which method comprises the additional step of storing the concentrated LDL solution at a temperature of between 1° and 10°C in the dark, and under liquid nitrogen, argon or an inert gas.

14. A concentrated LDL solution which does not contain a measureable lipid peroxidation.

Europäisches
Patentamt

European
Patent Office

Office européen
des brevets

# EUROPEAN SEARCH REPORT

Application Number

EP 11 18 5180

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MCDOWELL IFW ET AL: "A rapid method for measurement of the susceptibility to oxidation of low-density lipoprotein", ANNALS OF CLINICAL BIOCHEMISTRY, vol. 32, no. 2, 1995, pages 167-174, XP008149749, * page 167 - page 168 * | 1-14 | INV. C07K14/775 |
| X,D | VIEIRA OV ET AL: "Rapid Isolation of Low Densitiy Lipoproteins in a Concentrated Fraction Free From Water-soluble Plasma Antioxidants", JOURNAL OF LIPID RESARCH, vol. 37, 1996, pages 2715-2721, XP002671212, | 14 | |
| Y | * abstract * * page 2716 - page 2718 * | 1-13 | |
| X | WO 2005/095986 A2 (AREXIS AB [SE]; BJURSELL KARL GUNNAR [SE]; NILSSON JEANETTE ANNIKA [SE] 13 October 2005 (2005-10-13) | 14 | |
| Y | * page 13, line 40 - page 14, line 4 * | 1-13 | **TECHNICAL FIELDS SEARCHED (IPC)**<br>C07K |
| X | WO 2010/136546 A1 (UNIV BRUXELLES [BE]; SOUBHYE JALAL [BE]; DUFRASNE FRANCOIS [BE]; VAN A) 2 December 2010 (2010-12-02) * paragraphs [0058], [0094] - [0096]; claims 14,15; table 3 * | 14 | |
| X | EP 1 223 177 A1 (AGRONOMIQUE INST NAT RECH [FR]; ENVT TOULOUSE [FR]) 17 July 2002 (2002-07-17) | 14 | |
| A | * claims 1-3,8; figure 1 * | 1-13 | |

The present search report has been drawn up for all claims

| Place of search | Date of completion of the search | Examiner |
|---|---|---|
| The Hague | 9 March 2012 | Schmidt, Harald |

EPO FORM 1503 03.82 (P04C01)

**ANNEX TO THE EUROPEAN SEARCH REPORT
ON EUROPEAN PATENT APPLICATION NO.**                    EP 11 18 5180

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2012

| Patent document cited in search report | | Publication date | Patent family member(s) | | Publication date |
|---|---|---|---|---|---|
| WO 2005095986 | A2 | 13-10-2005 | AT | 447714 T | 15-11-2009 |
| | | | EP | 1735625 A2 | 27-12-2006 |
| | | | US | 2008262108 A1 | 23-10-2008 |
| | | | US | 2012053114 A1 | 01-03-2012 |
| | | | WO | 2005095986 A2 | 13-10-2005 |
| WO 2010136546 | A1 | 02-12-2010 | AU | 2010251930 A1 | 24-11-2011 |
| | | | EP | 2435043 A1 | 04-04-2012 |
| | | | WO | 2010136546 A1 | 02-12-2010 |
| EP 1223177 | A1 | 17-07-2002 | AT | 425185 T | 15-03-2009 |
| | | | DK | 1223177 T3 | 06-07-2009 |
| | | | EP | 1223177 A1 | 17-07-2002 |
| | | | ES | 2323854 T3 | 27-07-2009 |
| | | | FR | 2819264 A1 | 12-07-2002 |
| | | | PT | 1223177 E | 17-06-2009 |

EPO FORM P0459

For more details about this annex : see Official Journal of the European Patent Office, No. 12/82

EP 2 581 386 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHUNG, B. H. ; J. P. SERGREST ; M. J. RAY ; J. D. BRUNZELL ; J. E. HOKANSON ; R. M. KRAUSS ; K. BEAUDRIE ; J. T. CONE.** Single vertical spin density gradient ultracentrifugation. *Methods Enzymol.,* 1986, vol. 128, 181-209
- **HAVEL, R.J. ; EDER, H.A. ; BRAGDON, J.H.** The distribution and chemical composition of ultracentrifugally separated lipoproteins in human serum. *J. Clin. Invest.,* 1955, vol. 34, 1345-1353
- **KLEINVELD H. A. ; HAK-LEMMERS H. L. M. ; STALENHOEF A. F. H. ; DEMACKER P. N. M.** Improved measurement of low-density lipoprotein susceptibility to copper-induced oxidation: Application of a short procedure for isolating low-density lipoprotein. *Clin Chem,* 1992, vol. 38, 2066-2072

- **LOWRY, 0. H. ; N. J. ROSEBROUGH ; A. L. FARR ; R. J. RANDALL.** Protein measurement with the Folin phenol reagent. *J. Biol. Chem.,* 1951, vol. 193, 265-275
- **MIRONOVA M ; VIRELLA G ; LOPES-VIRELLA M. F.** Isolation and Characterization of Human Antioxidized LDL Autoantibodies. *Arteriosclerosis, Thrombosis, and Vascular Biotogy,* 1996, vol. 16, 222-229
- **VIEIRA V. OTILIA ; LARANJINHA, JOÃO A. N. ; MADEIRA M. C. VITOR ; ALMEIDA M. LEONOR.** Rapid Isolation of Low Densitiy Lipoproteins in a Concentrated Fraction Free From Water-soluble Plasma Antioxidants. *Journal of Lipid Resarch,* 1996, vol. 37, 2715-2721